# EUROPEAN PATENT APPLICATION

(11) **EP 4 226 874 A1**
(43) Date of publication of application: **16.08.2023**
(21) Application number: 22155806.7
(22) Date of filing: 09.02.2022
(51) Int. Cl.: A61B 17/225, A61B 17/00

(54) **ULTRASOUND AND/OR SHOCK WAVE DEVICE WITH HEXAPOD PLATFORM MOUNTED SOURCE**

(71) Applicant: Storz Medical AG, 8274 Tägerwilen (CH)
(72) Inventor: Storz, Rafael, 8280 Kreuzlingen (CH); Marlinghaus, Ernst, 8598 Bottighofen (CH)
(74) Representative: Lohr, Jöstingmeier & Partner

(57) **Abstract**

A wave and/or ultrasound device, e.g., a lithotripter comprises a patient table and an ultrasound and/or shockwave source below the patient table, the table defining a table plane. The ultrasound and/or shockwave source is coupled to a hexapod drive and configured for a 6 degrees of freedom movement.

## Description

### Field of the invention

The invention relates to an extracorporeal ultrasound and/or shock wave therapy system, e.g., a lithotripsy system or Lithotripter for non-invasive treatment of stones, like e.g. kidney stones, urinary stones, gallstones or other calculi within a mammal's body using acoustic pulses, or other applications of an extracorporeal ultrasound and/or shock wave therapy system where the treatment area may be in motion during treatment.

### Description of the related art

A device and a method for localizing stones and for targeting a shockwave source of a lithotripter is disclosed in EP 2 340 781 A1. Here a shockwave generator, a patient table and an ultrasound targeting device are provided with optical markers for tracking with a pair of infrared cameras. The body or parts of the body of a patient move slightly during breathing. This causes kidneys and other organs to move slightly such that they may move out of the focal volume of a shockwave source.

DE 36 21 935 discloses a lithotripter which is synchronized to the signal of a respiration sensor. This allows only for short treatment periods with a comparatively low repetition frequency corresponding to the respiration frequency.

### Summary of the invention

The problem to be solved by the invention is to provide an ultrasound and/or shock wave device, which allows treatment of organs or areas within organs independent of respiration or other movements. The shock wave or ultrasound device should be comparatively robust and should provide a high degree of freedom in compensation of movements.

Solutions of the problem are described in the independent claims. The dependent claims relate to further improvements of the invention.

In an embodiment, a shock wave and/or ultrasound device, which may be a lithotripter includes an ultrasound and/or shockwave source and may further include a patient table. The ultrasound and/or shockwave source may be of any type suitable for generating shock waves. It may include a shock wave generator and/or transducer, which may include at least one of a coil, a spark gap or a Piezo transducer. The shock wave generator/transducer may be partially enclosed by a reflector. Depending on the type of transducer, the reflector may have a parabolic or half-elliptic shape. The ultrasound and/or shockwave source may have a focal volume which is distant from the ultrasound and/or shockwave source and normally around a center axis of the ultrasound and/or shockwave source. The focal volume may be defined as a volume, where the maximum shock wave intensity is maintained with a deviation of maximal -3 dB or -6 dB. If the focal volume is defined with a 6 dB deviation, the pressure at the limit of the zone is half of the maximum pressure inside the zone. The focal volume may have an elliptical shape with a length in an axial direction (defined by the center axis) of the ultrasound and/or shockwave source axis of 10 to 15 cm and a diameter between 5 and 15 mm. The focal volume normally is spaced from the shock wave generator and/or transducer.

The patient table may have a basically planar surface defining a longitudinal axis. It is configured for accommodating a patient. The ultrasound and/or shockwave source may be mounted below the patient table. In general, a shockwave source may be mounted in alternative ways, e.g., on a stand or support.

Due to movements of a patient's body, e.g., by respiration or heartbeat, an object to be treated may move out of the focal volume. For example, a movement of the chest causes a movement of the kidneys in a cranio-caudal direction, which is approximately parallel to the surface of the patient table accommodating the patient. The amplitude of displacement of the kidneys normally is in the range of 20 to 40 mm. As the center axis of a normal shock wave generator is approximately orthogonal to the surface of the patient table, a kidney stone may easily move out of the focal volume. The respiration frequency of an anesthetized person may be in the range of 0.3 to 0.5 Hz.

In an embodiment, an ultrasound and/or shockwave source is supported by and/or suspended by a hexapod drive which may also be called a hexapod platform. Such a hexapod platform also is called a Stewart platform. Basically, it is a type of parallel manipulator or parallel robot that has six linear actuators, which may be hydraulic or pneumatic jacks or electric linear actuators. Examples of electric linear actors are motors coupled to a belt or a spindle to perform a linear movement. These linear actuators are attached in pairs to three positions at a base, crossing over to three mounting positions at the ultrasound and/or shockwave source. Each connection of a linear actuator to either the base or the ultra-sound and/or shockwave source may include a universal joint, also called a cardan joint or a ball joint. By variation of the length of the linear actuators, the ultrasound and/or shockwave source can be moved in six degrees of freedom with respect to the base. There are three degrees of translation and three degrees of rotation. Although the use of six linear actuators is preferred, a lower number of actuators may be used, e.g., like in a delta robot with three actuators.

The hexapod drive allows to adjust the position of an ultrasound and/or shockwave source to a patient's body. Positioning of the ultrasound and/or shockwave source may be done automatically or by manual control. An automatic control may allow quick adjustment and it may also allow to store and retrieve preconfigured settings.

Such a hexapod drive is a very robust and mechanical stiff support or suspension of the ultrasound and/or shockwave source. Therefore, it can withstand high forces from the patient body, the weight of the ultrasound and/or shockwave source and dynamic loads which occur when shockwave pulses are generated. Further, a hexapod drive can be moved quickly. Therefore, a stable positioning of the ultrasound and/or shockwave source is achieved providing the additional ability to quickly correct deviations and movements by the patient.

A hexapod drive may allow movement in all 6 degrees of freedom. This allows for a precise adjustment of the position of the focal volume of the ultrasound and/or shockwave source and/or the path of the ultrasound and/or shockwave through the body of the patient. The patient table may be positioned for a slow and coarse adjustment in 3 degrees of translation for larger distances whereas the hexapod drive provides a comparatively quick adjustment in 3 degrees of translation and 3 degrees of rotation. The movement distances of the table may be larger than the movement distances of the hexapod drive.

In an embodiment, the ultrasound and/or shockwave source is driven by a hexapod drive to compensate for movements of the patient during treatment. By the hexapod drive, the ultrasound and/or shockwave source may be tiltable around at least one tilt axis which may be approximately parallel to a plane defined by a surface of a patient table, where a deviation in parallelism may be of +/- 30°. Further, the tilt axis may be orthogonal to a longitudinal axis of the patient table, where deviation in orthogonality of +/- 30° may be possible. The maximum angle of tilt may be in a range between 1° and 20°. It may be in a range between 1° and 10° or between 2° and 5°. A number of tests have shown that a typical angle of 2° is sufficient to track typical stone movements, whereas for larger movements an angle of 4° may be sufficient with given typical focal distances measured from the outer rim of a reflector which may be in a range between 100 mm and 200 mm, e.g. 140 mm or 180 mm. Tilting may be synchronized to the motion of the patient body, which may be sensed by a motion sensor or by a respiration sensor. Such a sensor may also be a camera. A respiration sensor may be any sensor configured for at least one of a chest movement of the patient due to respiration, a change of chest volume and/or size of the patient due to respiration, a change of position of an internal organ like kidney or heart by using ultrasound-imaging, a change of center of gravity of the patient and/or an organ of interest, which may be a kidney to be treated. Herein, for simplicity, such a sensor may be called a respiration sensor. The signal of the movement sensor/respiration sensor may be amplified with a variable gain. Further, an offset may be added to generate a tilt control signal defining the tilt angle. By this, a coupling of the tilt angle to the body movement and therefore to the movement of the kidney stone may be achieved.

The base may be standing on a floor directly or by a stand or it may be attached to a floor. The base may also hold the table.

As the position of the focal volume moves in synchronicity with the kidney stone, generating of shock wave pulses at any time will result in a high energy coupling into the stone and a high treatment efficiency.

For heart treatment, respiration and heartbeat may be considered. This may require a tilting movement about two different axes with different frequencies. Basically, all parts of a body may be treated by compensating any movement by a tilting movement.

A fine adjustment of the amplitude of the tilting movement may either be made manually by a user who may watch the imaging system and an indication of the focal volume. Further, adjustment of the amplitude may be made automatically, for example by a computer system, analyzing the images of the imaging system.

A display may be provided indicating an image from an imaging system, like an x-ray image or and ultrasound image, which may further indicate the focal volume. Based on this, a user may estimate the quality of adjustment and treatment. Additional image analysis may provide a figure of merit as feedback for the user with graphical indicators (bar graphs, colored feedback etc.).

Due to the body movement or respiration tracking of the ultrasound and/or shockwave source, any shock wave repetition frequency, may be selected independent of the respiration frequency.

In an embodiment, the ultrasound and/or shockwave source may be rotated around the center axis about an angle in a range between +/- 2° to +/- 20°. The range may be between +/- 5° and +/-15°. In a typical application, a range of +/-10° would meet most requirements. Usually, kidneys and kidney stones have a slightly outward movement in addition to the movement parallel to the patient table, which may be compensated by such a rotation around the center axis. In a very simple embodiment, there may be only two discrete angle settings, for example at + 10° and at - 10°, one for the left kidney and the other for the right kidney, which would suit most patients. In another embodiment, a larger number of positions may be provided.

The basic concept explained herein at the example of a kidney stone may also be applied to a urethra stone or other concrements in a body.

A method of focusing an ultrasound and/or shockwave source on a hexapod platform to a kidney stone comprises the steps of:
a) receiving a respiration signal indicative of a respiration,
b) amplifying the signal and optionally adding an offset,
c) tilting an ultrasound and/or shockwave source about an angle proportional to the signal.

The tilt axis may be an axis in a plane essentially parallel to a plane of a patient table and essentially orthogonal to a longitudinal axis of the patient table, including deviations in parallelism and/or orthogonality of +/- 30°.

The method may further include the steps of rotating the ultrasound and/or shockwave source around its center axis or a fixed angle before performing the steps a to c.

### Description of Drawings

In the following the invention will be described by way of example, without limitation of the general inventive concept, on examples of embodiment with reference to the drawings.
- Figure 1: shows an embodiment.
- Figure 2: shows a side view.
- Figure 3: shows an embodiment of a therapy system.
- Figure 4: shows focal volume positions with tilting.
- Figure 5: shows a top view.
- Figure 6: shows a block diagram.

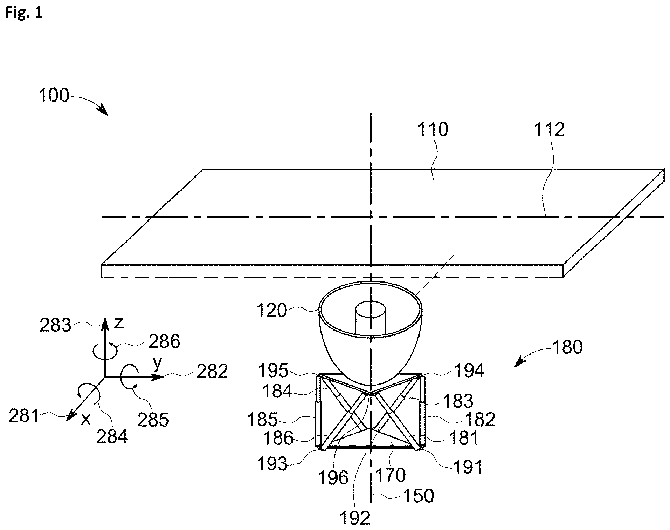

In Figure 1, a first embodiment is shown. A shock wave or ultrasound device, which may be a lithotripter 100 may include a patient table 110 and an ultra-sound and/or shockwave source 120. The ultrasound and/or shockwave source 120 may be arranged below the patient table 110, such that a patient (not shown here) may be accommodated on top of the patient table. The patient table may have a longitudinal axis 112. There may be a hole or cutout in the patient table at the position of the ultrasound and/or shockwave source.

The ultrasound and/or shockwave source 120 is supported by and/or suspended by a hexapod drive 180. Basically, it is a type of parallel manipulator or parallel robot that has six linear actuators 181 - 186, which may be hydraulic or pneumatic jacks or electric linear actuators. These linear actuators are connected in pairs to three mounting positions 191, 192, 193 at a base 170, crossing over to three mounting positions 194, 195, 196 at the ultrasound and/or shockwave source 120. Each connection of a linear actuator to either the base or the ultra-sound and/or shockwave source may include a universal joint, also called a cardan joint or a ball joint. By variation of the length of the linear actuators, the ultrasound and/or shockwave source can be moved in six degrees of freedom with respect to the base. There are three degrees of translation, parallel to at least one X-axis 281, Y-axis 282, Z-axis 283 and three degrees of tilt or rotation including a first tilt 284 around an axis parallel to X-axis 281, a second tilt 285 around an axis parallel to Y-axis 282, a third tilt 286 around an axis parallel to Z-axis 283.

Further, the ultrasound and/or shockwave source 120 may have a center axis 150.

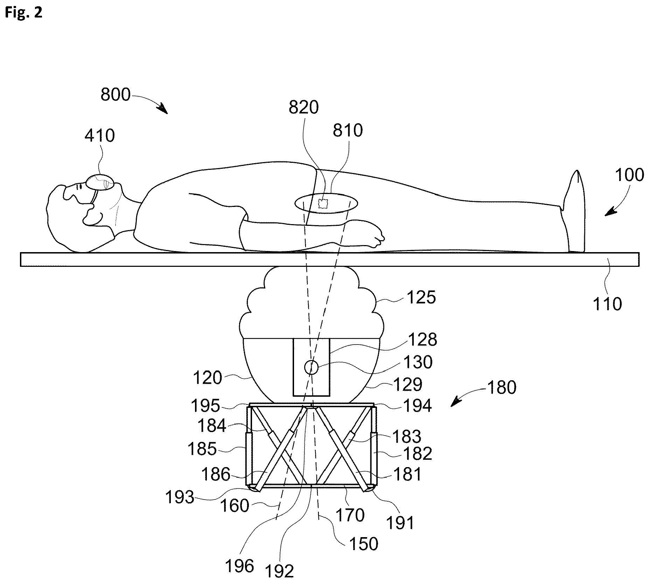

Figure 2 shows a side view. In this figure, a patient 800 is positioned on top of the patient table 110. The patient may have a kidney 810 with a kidney stone 820. Below the table 110, the ultrasound and/or shockwave source 120 is shown in a sectional view. It may have a shock wave generator 128 which may be a coil as shown herein and which is at least partially enclosed by a reflector 129. Further, the center axis 150 is marked as a dashed line and a tilted center axis 160 of a tilted state of the ultrasound and/or shockwave source is marked as a second dashed line. Normally, the interior of the reflector and the space between the source and the patient is filled with a liquid like water or another shockwave conducting medium. To contain the water within the volume, a coupling bellow 125 may be provided.

The patient may have a respiration sensor 410, configured to produces a respiration signal.

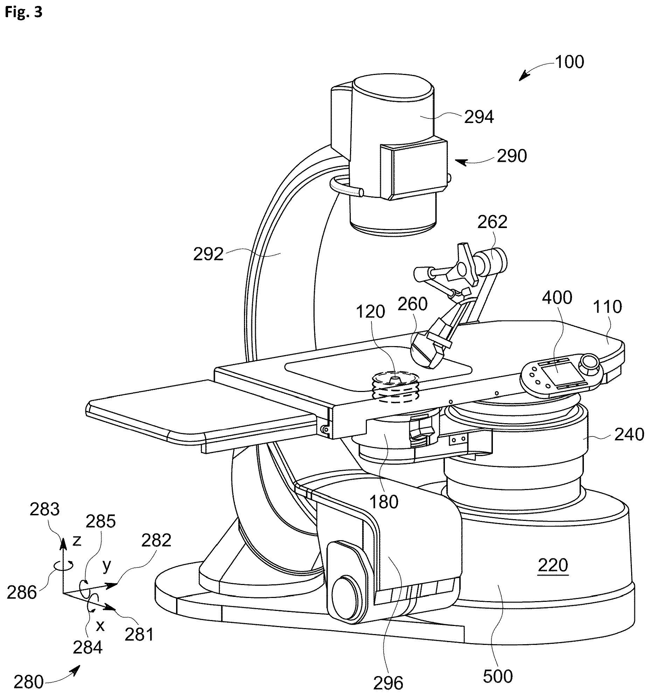

Figure 3 shows an embodiment of a therapy system. Here, a lithotripsy system is shown for example. The invention works with any ultrasound and/or shockwave treatment system.

An extra-corporal ultrasound and/or shockwave lithotripsy system for non-invasive treatment of stones 100 comprises a patient table 110, an ultra-sound and/or shockwave source 120.

An ultrasound and/or shockwave source 120 is mounted to a hexapod drive 180 and which may further be held by a stand 220. The hexapod drive 180 allows fine positioning of the ultrasound and/or shockwave source 120 in multiple axes relative to the patient table 110 and therefore relative to the patient 800 (not shown in this figure). The ultrasound and/or shockwave source 120 has a focal volume which moves together with the source. In an embodiment the distance of the focal volume to the source may be modified.

The patient table 110 is based on a stand 220 which may stand on a floor and which may include a solid platform 500. The table 110 may be held by a positioning device 240 to move the table relative to the ultrasound and/or shockwave source 120 together with the hexapod drive 180. A movement of the patient table may be coarse positioning which may be limited to a displacement in 3 axes.

The table 110 has a flat surface for accommodating a patient 800 which is not shown here.

There may be an ultrasound transducer 260, also called targeting device, for locating the stone. The area around the stone may be the target area. The ultra-sound transducer 260 may be held at the table by an arm 262 and it may be movable together with the table, such that it maintains a constant spatial relationship with the table, and therefore with the patient and its stone. In another embodiment, an ultrasound transducer for locating the stone may be integrated into the source, for example arranged coaxially at the center of the source.

An X-ray device 290, acting a targeting device, may be provided. It may have an X-ray tube at a C-arm 292 and may be used for localizing a stone (a target area) in the body of a patient additionally or alternatively to the ultrasound transducer 260.

To describe the relative movement, a cartesian coordinate system 280 may be used. There is an y-axis 282, which may be a longitudinal axis through the center of the table. Furthermore, there is a x-axis 281 orthogonal to the y-axis and in the plane of the table surface. A z-axis 283 is orthogonal to the plane of the table surface and therefore orthogonal to the x-axis and the y-axis. There may also be a first rotation 284 around the x-axis, a second rotation 285 and a third rotation 286 around the z-axis. A positive rotation may be a clockwise rotation in a view along a positive axis.

A hexapod drive may allow movement in all these 6 degrees of freedom. This allows for a precise adjustment of the position of the focal volume of the ultra-sound and/or shockwave source 120. The patient table may be positioned for a slow and coarse adjustment in 3 degrees of translation for larger distances whereas the hexapod drive provides a comparatively quick adjustment in 3 degrees of translation and 3 degrees of rotation. The movement distances of the table may be larger than the movement distances of the hexapod drive.

For control of the movement of the ultrasound and/or shockwave source 120 relative to the patient table 110, a control panel 400 may be provided.

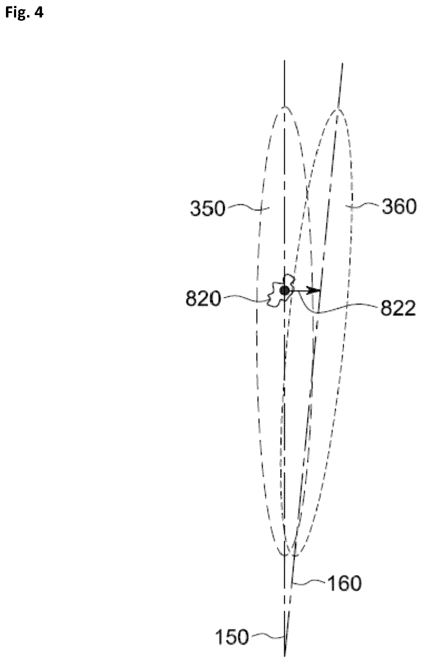

Basically, the hexapod drive allows for a precise adjustment of the position of the focal volume of the ultrasound and/or shockwave source and/or the path of the ultrasound and/or shockwave though the body of the patient. In an embodiment, the ultrasound and/or shockwave source is driven by a hexapod drive to compensate for movements of the patient during treatment. Such an embodiment is explained in more detail in figures 4, 5 and 6.

Figure 4 shows focal volume positions with tilting in a side view. On the left side, a first focal volume 350 is shown around the center axis 150 in a first state. When tilting the ultrasound and/or shockwave source about an angle, this focal volume is displaced to second focal volume 360 around a tilted center axis 160. In this figure, a tilt angle of approximately 4° is shown. It can be seen that the first focal volume from a normal state and the second focal volume from a tilted state together cover roughly twice the volume at the center of the focal volumes. A kidney stone 820, which may be centered to the center axis 150 in a first state, may move by respiration movement in a direction 822 to a second position, which then may be close to the tilted center axis 160. As the tilt of the ultrasound and/or shockwave source is synchronized with the respiration movement and therefore with the movement of the kidney 810 and with the movement of the kidney stone 820, the kidney stone is always approximately at the center axis of the ultrasound and/or shockwave source and therefore within the focal volume.

This ensures a high energy coupling into the stone at any time.

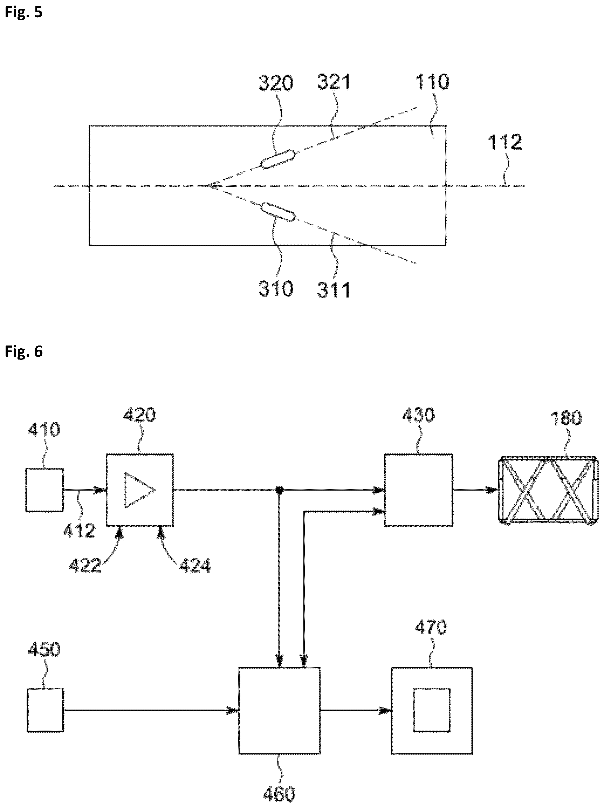

Figure 5 shows a top view. As the kidneys not only move roughly parallel to the longitudinal axis 112 of the body and therefore of the patient table, but also move slightly sidewards, the ultrasound and/or shockwave source may be rotated around the center axis 150 in a first direction, such that the tilt results in movement in a first tilt direction 311 under an angle to the longitudinal axis 112.

Alternatively, the ultrasound and/or shockwave source may be rotated into an opposing second position, resulting in a tilting movement in a second tilt direction 321. The focal volume covered during the tilt movement is indicated with reference number 310 for the first tilt direction 311 and with reference number 320 for the second tilt direction 321.

Figure 6 shows a block diagram. A respiration sensor 410 produces a respiration signal or a signal related to respiration. Such a respiration signal may be a signal indicating the air flow into the body and/or out of the body, and/or a signal related to a movement of the body. For example, a sensor measuring the circumference of the chest may be used to generate a signal for such an indication. The signal is coupled into a signal amplifier 420 which may further receive a gain signal 422 and an offset signal 424. The gain signal and the offset signal may either be manually set by an operator and/or may be set by a controller/computer 460.

This signal allows to modify the amplitude of the signal received by the respiration sensor and to add an offset if desired. The output signal of the signal amplifier 420 may be fed to a computer/controller 460. It may also be fed to a motor controller 430 which generates a control signal to the hexapod 180. The motor controller 430 may include at least one sensor for position and/or orientation of the hexapod to provide a positioning feedback. Based on the signals of the at least one sensor, the controller may adjust the position and/or orientation of the hexapod as required by control signals received.

The hexapod 180 may tilt the ultrasound and/or shockwave source. In an embodiment, there may be a linear relationship between the input signal into the motor controller 430 and the tilt angle, further resulting in a linear relationship between the respiration sensor signal and the tilt angle. To prevent excessive tilt angles, the motor controller 430 and/or the signal amplifier 420 may have a limiter to limit the output signal.

The controller/computer 460 may also receive a signal from an imaging system 450 which may be used to evaluate the position of the kidney stone. The controller/computer 460 may further be connected to a display 470 at which it may display an image from the imaging system 450 and/or an indication of the focal volume. The focal volume may be displayed in the same image, thus giving an indication of the overlapping of the focal volume with the kidney stone. This would allow to manually adjust the gain signal 422 and the offset signal 424 for best matching during respiration of the patient. The controller/computer 460 may also be connected to the motor controller 430 to control the hexapod 180 e.g., based on the respiration sensor signal and/or a stone position derived from an image.

### List of reference numerals

- 100: lithotripter
- 110: patient table
- 112: longitudinal axis
- 120: ultrasound and/or shockwave source
- 125: coupling bellow
- 128: shock wave generator
- 129: reflector
- 150: center axis
- 151: rotation direction
- 160: tilted center axis
- 170: base
- 180: hexapod drive
- 181-186: linear actuators
- 191-193: mounting positions at base
- 194-196: mounting positions at ultrasound and/or shockwave source
- 220: stand
- 240: positioning device
- 260: ultrasound transducer
- 262: arm for ultrasound transducer
- 280: first cartesian coordinate system
- 281: x-axis
- 282: y-axis
- 283: z-axis
- 284: tilt around the x-axis
- 285: tilt around the y-axis
- 286: tilt around the z-axis
- 290: X-ray device
- 292: C-arm
- 294: X-ray detector
- 296: X-ray tube
- 310: first focal volume coverage
- 311: first tilt direction
- 320: second focal volume coverage
- 321: second tilt direction
- 350: first focal volume
- 360: second focal volume
- 400: control panel
- 410: movement sensor/respiration sensor
- 412: movement sensor/respiration sensor signal
- 420: signal amplifier
- 422: gain signal
- 424: offset signal
- 430: motor controller
- 450: imaging system
- 460: controller/computer
- 470: display
- 500: base
- 800: patient
- 810: kidney
- 820: kidney stone
- 822: movement of kidney stone

## Claims

1. A shock wave and/or ultrasound device (100) comprising an ultrasound and/or shockwave source (120) and a base (170),
the ultrasound and/or shockwave source (120) being suspended on a hexapod drive (180) which includes six linear actuators (181-186) and which are attached in pairs to three positions (191-193) at the base (170), crossing over to three mounting positions (194-196) at the ultrasound and/or shockwave source (120).

2. The shock wave and/or ultrasound device (100) according to claim 1, **characterized in, that**
each connection of a linear actuator (181-186) to either the base (170) or the ultrasound and/or shockwave source (120) includes a universal joint or a ball joint.

3. The shock wave and/or ultrasound device (100) according to any of the preceding claims,
**characterized in, that**
the linear actuators (181-186) include at least one of hydraulic or pneumatic jacks or electric linear actuators.

4. The shock wave and/or ultrasound device (100) according to any of the preceding claims,
**characterized in, that**
the hexapod drive (180) being coupled to a movement sensor (410) and configured for a tilting movement (132) as a function of a movement sensor signal (412) from the movement sensor (410).

5. The shock wave and/or ultrasound device (100) according to any of the preceding claims,
**characterized in, that**
the tilting movement (132) is limited to an angle less than one of 20°, 10°, 5° 4° 2°.

6. The shock wave and/or ultrasound device (100) according to any of the preceding claims,
**characterized in, that**
the shock wave and/or ultrasound device (100) is a lithotripter comprising a patient table (110), and the movement sensor (410) is at least one of a respiration sensor, a camera or a heart pulse sensor.

7. The shock wave and/or ultrasound device (100) according to the preceding claim,
**characterized in, that**
the respiration sensor (410) is coupled to a patient (800), the patient being positioned on the patient table.

8. The shock wave and/or ultrasound device (100) according to any of the preceding claims,
**characterized in, that**
the ultrasound and/or shockwave source (120) is configured to generate shock waves propagating in a direction to the patient table (110) and having a focal volume (350) above the patient table (110).

9. The shock wave and/or ultrasound device (100) according to the preceding claim,
**characterized in, that**
the ultrasound and/or shockwave source (120) is configured to move the focal volume (350) by tilting parallel to the longitudinal axis (112) of the patient table (110) with a deviation caused by rotation.

10. The shock wave and/or ultrasound device (100) according to any of the preceding claims,
**characterized in, that**
the respiration sensor (410) is configured for sensing at least one of
- a respiration air flow in and/or out of the patient (800) coupled to a patient (800),
- a chest movement of the patient (800) due to respiration, and
- a change of chest volume and/or size of the patient (800) due to respiration
- a change of position of an internal organ like kidney or heart by using ultrasound-imaging,
- a change of center of gravity of an organ of interest.

11. The shock wave and/or ultrasound device (100) according to any of the preceding claims,
**characterized in, that**
at least one imaging system (450) is provided and configured for generating images of a region of a patient.

12. The shock wave and/or ultrasound device (100) according to any of the preceding claims,
**characterized in, that**
at least one controller/computer (460) is provided and configured to at least one of:
- calculating an optimal tilt angle and/or rotation angle,
- visualizing on a screen the position of an organ (810) and/or a stone (820) together with a focal volume (350),
- visualizing the movement of a focal volume (350) due to tilt,
- visualizing the total focal volume (310) covered by tilt.

13. A method of operating a shock wave and/or ultrasound device (100) having an ultrasound and/or shockwave source (120) including a hexapod drive (180) further including the steps of:
a) receiving a respiration sensor signal (412) from a respiration sensor (410),
b) amplifying the signal
c) tilting the ultrasound and/or shockwave source (120) by the amplified signal
and the optional further steps of:
d) calculating an optimal tilt angle and/or rotation angle,
e) visualizing on a screen the position of an organ (810) and/or a stone (820) together with a focal volume (350),
f) visualizing the movement of a focal volume (350) due to tilt,
g) visualizing the total focal volume (310) covered by tilt.

14. The method of the previous claim wherein step b includes at least one of the steps of:
b1) modifying the amplitude of the signal,
b2) adding an offset to the signal.
